# EUROPEAN PATENT APPLICATION

(11) **EP 4 324 843 A1**
(43) Date of publication of application: **21.02.2024**
(21) Application number: 22191096.1
(22) Date of filing: 18.08.2022
(51) Int. Cl.: C07K 14/34, C12N 1/20, C12N 15/77, C12N 15/62

(54) **PRODUCTION OF CRM197**

(71) Applicant: SenseUp GmbH, 52428 Jülich (DE)
(72) Inventor: SCHAUMANN, Georg, 52425 Jülich (DE)
(74) Representative: Stolmár & Partner Patentanwälte PartG mbB

(57) **Abstract**

The invention relates to a method for producing a protein in a Corynebacterium cell, wherein the protein is a non-toxic variant of diphtheria toxin and wherein a signal peptide having at least 90% sequence identity to SEQ ID NO: 1 or SEQ ID NO: 5 is fused to the protein.

## Description

### Field of the invention

The invention relates to methods for the production of CRM197 as well as cells capable of expressing CRM197.

### Background of the invention

Diphtheria toxin (DT) is a protein toxin produced and secreted by pathogenic strains of *Corynebacterium diphtheriae.* The pathogenic strains contain a bacteriophage lysogen that carries the toxin gene. DT is an ADP-ribosylating enzyme that is secreted as a proenzyme containing 535 amino acid residues and is processed by trypsin-like proteases into two fragments (A and B). Fragment A uses NAD as a substrate, catalyzing the cleavage of the N-glycosidic bond between the nicotinamide ring and the N-ribose, additionally mediating the covalent transfer of the ADP-ribose to the modified histidine at amino acid position 715 of the elongation factor EF-2. Concomitantly EF-2 is inactivated halting protein synthesis and resulting in cell death. The B fragment mediates binding to receptors on the host cell surface and further promotes pH-dependent transfer of fragment A into the cell.

CRM197 is a non-toxic mutant of the DT, containing a single amino acid substitution of glutamic acid for glycine at position 52 in the amino acid sequence (G52E). This substitution renders the protein enzymatically inactive towards NAD and non-toxic. CRM197 has been found to be an ideal carrier for conjugate vaccines against encapsulated bacteria. In a conjugate vaccine CRM197 is covalently linked to poorly immunogenic and T-cell independent capsular polysaccharides, thereby creating conjugate antigens that are highly immunogenic and result in long-lived immunity against the antigen(s).

Vaccines containing CRM197 as a carrier protein include Menveo^{®}, a conjugate vaccine against serogroups A-C-W135-Y of *Neisseria meningitidis,* Menjugate^{®} and Meningitec^{®} against meningitidis, Vaxem-Hib^{®} and HibTITER^{®} against *Haemophilus influenza* type B and Prevnar^{®} against pneumococcal bacterial infection.

Commonly, *Corynebacterium diphtheriae* C7 is used for the commercial manufacture of CRM197, wherein CRM197 is expressed from multiple lysogens of the beta-phage and finally released in the media during fermentation. The yield of CRM197 is low, ranging from tens of mg/L to 200 mg/L, and requires biosafety level 2 facilities, which increases the cost price of CRM197 and availability for vaccination programs in developing countries.

Using *E. coli* as heterologous producer has not led to the desired production of high amounts of soluble protein. CRM197 has also been expressed in a proprietary *Psuedomonas fluorescence* strain, wherein the protein is secreted into the periplasm of the cell. However, this complicates the purification of the recombinant CRM197 protein. In addition, gram-negative bacteria like E. *coli* and P. *fluorescence* contain lipopolysaccharides (LPS), generally referred to as endotoxins, which are pyrogenic in humans and other mammals. These endotoxins complicate product purification because the end product should be endotoxin-free.

### Objective technical problem

Therefore, there remains a need for method of producing CRM197 that is both safe and effective and requires little additional purification.

### Summary of the invention

The problem is solved by a method for producing a protein in a Corynebacterium cell, wherein the protein is a non-toxic variant of diphtheria toxin and wherein a signal peptide having at least 90% sequence identity to SEQ ID NO: 1 or SEQ ID NO: 5 is fused to the protein.

The problem is further solved by a Corynebacterium cell capable of expressing a non-toxic variant of diphtheria toxin and wherein a signal peptide having at least 90% sequence identity to SEQ ID NO: 1 or SEQ ID NO: 5 is fused to the non-toxic variant of diphtheria toxin.

In another aspect, the problem is solved by a DNA molecule encoding a non-toxic variant of diphtheria toxin, the DNA molecule comprising a part having at least 90 % sequence identity to SEQ ID NO: 4.

### Brief description of the figures

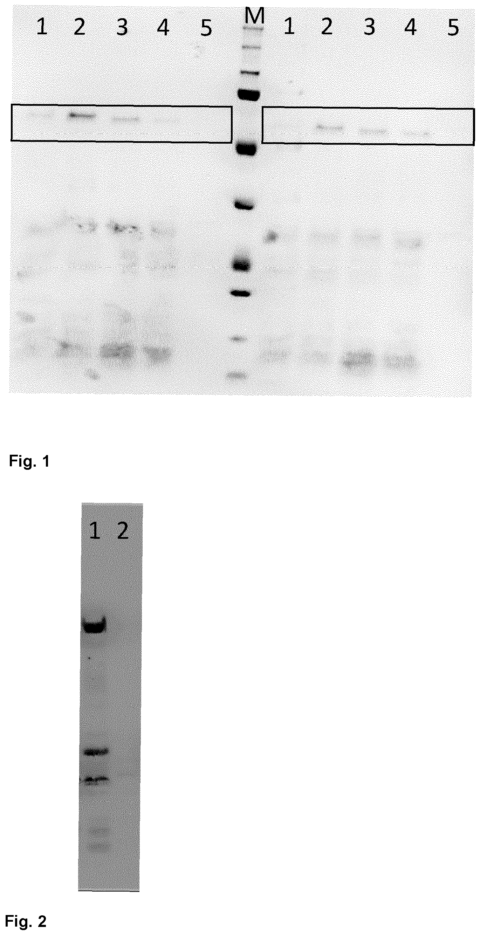

**Fig. 1** shows a Western Blot (WB) using Anti-Diphtheria Toxin Antibody to visualize the CRM197 protein via chemiluminescence of culture supernatants from *Corynebacterium* cells producing CRM197 and control culture which does not express CRM197. In lane M, the PageRuler^{™} Plus Prestained Protein Ladder was loaded. To the left of the marker lane, samples of cultures were loaded that were induced with 1 mM Isopropyl β- d-1-thiogalactopyranoside (IPTG), while on the right side of the marker lane cultures were induced with 0.2 mM IPTG. The rectangular box indicates the position of full length CRM197 (58 kDa) on the gel. 1: non-codon optimized CRM197 sequence with signal peptide 1 (Sp1_codonWT) 2: codon optimized sequence 1 of CRM197 SEQ ID NO: 3 combined with signal peptide 1 (Sp1_codon opt1) 3: codon optimized sequence 1 of CRM197 SEQ ID NO: 3 combined with signal peptide 2 (Sp2_codon opt1) 4: codon optimized sequence 2 of CRM197 (SEQ ID NO: 7) combined with signal peptide 2 (Sp2_codon opt2) 5: supernatant of C. *glutamicum* cell not expressing CRM197.
**Fig. 2** shows a Western Blot (WB) using Anti-Diphtheria Toxin Antibody to visualize the CRM197 protein via chemiluminescence. 1: Culture supernatant of *Corynebacterium* cell producing codon optimized sequence 1 of CRM197 SEQ ID NO: 3 with signal peptide 2 (Sp2_codon opt1) 2: Culture supernatant of *Corynebacterium* cell producing codon optimized sequence 3 of CRM197 (SEQ ID NO: 8) combined with signal peptide 2 (Sp2_codon opt3)

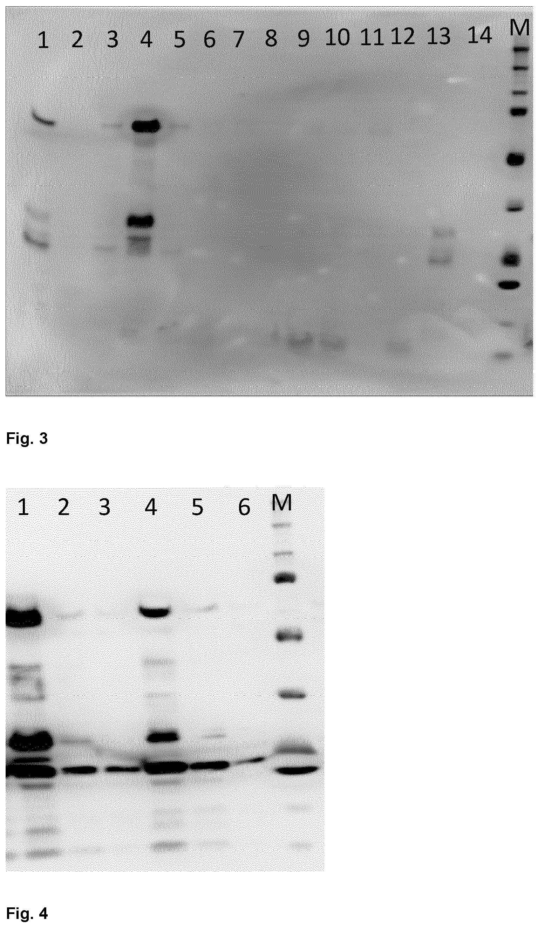

**Fig. 3** shows a Western Blot (WB) using Anti-Diphtheria Toxin Antibody to visualize the CRM197 protein via chemiluminescence of culture supernatants from the signal peptide library screening with *Corynebacterium glutamicum.* In lane M, the PageRuler^{™} Plus Prestained Protein Ladder was loaded. Lane 1 till 12 are culture supernatants from strains expressing different signal peptide combinations that have been fused with the CRM197 codonopt1 sequence SEQ ID NO: 3. Lane 4 depicts the CRM197 production when fused to the csn signal peptide SEQ ID NO: 1. Lane 13 depicts the CRM197 production when fused to its native secretion signal peptide from *Corynebacterium diphtheriae.* Lane 14 depicts supernatant from *Corynebacterium glutamicum* that does not contain the sequence for expressing CRM197.
**Fig. 4** shows a Western Blot (WB) using Anti-Diphtheria Toxin Antibody to visualize the CRM197 protein via chemiluminescence of culture supernatants from *Corynebacterium* cells producing CRM197 with different signal peptides fused to CRM197 codonopt1 sequence (SEQ ID NO: 3). Signal petide sequences from the following genes were used 1: *csn* B. *subtilis* (SEQ ID NO: 1) 2: cg1514 C. *glutamicum* 3: *nprE B. subtilis 4: cspA Corynebacterium ammoniogenes* (SEQ ID NO: 5) 5: CgR_0949 C. *glutamicum R* strain 6: *phoD B. subtilis.* In lane M, the PageRuler^{™} Plus Prestained Protein Ladder was loaded.

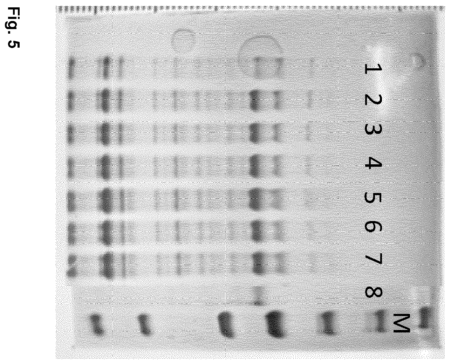

**Fig. 5** shows an SDS gel of supernatant samples taken at different time points from a 1 L fermentation process where *C.glutamicum* is expressing CRM197 codonopt1 sequence (SEQ ID NO: 3) fused to the *csn* signal peptide (SEQ ID NO: 1). 1: 8 hours after feed start 2: 24 hours after feed start 3: 28 hours after feed start 4: 32 hours after feed start 5: End feed 6: 3 hours after feed end 7: 7 hours after feed end 8: CRM197 standard from antikoerper-online. In lane M, the PageRuler^{™} Plus Prestained Protein Ladder was loaded.

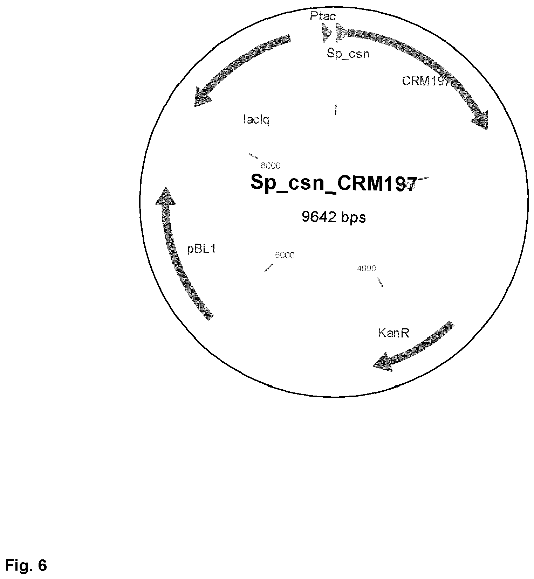

**Fig. 6** shows a plasmid map of the construct used for the expression of CRM197 protein fused to csn signal peptide under control of the Ptac promoter (SEQ ID NO: 4).

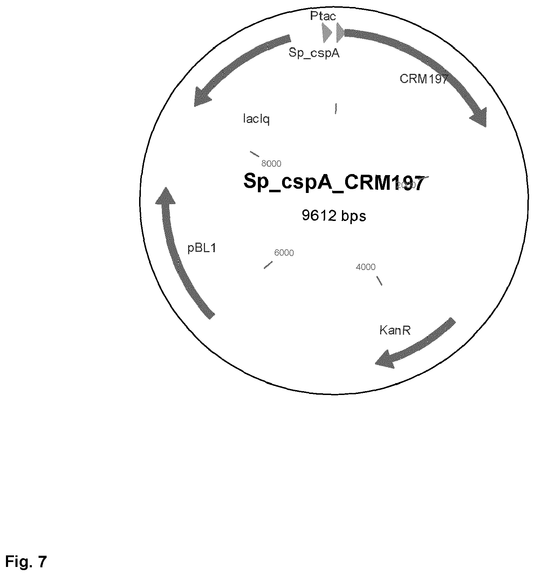

**Fig. 7** shows a plasmid map of the construct used for the expression of CRM197 protein fused to cspA signal peptide under the control of the Ptac promoter (SEQ ID NO: 6).

### Detailed description of the invention

In one aspect, the invention relates to a method for producing a protein in a Corynebacterium cell, wherein the protein is a non-toxic variant of diphtheria toxin and wherein a signal peptide having at least 90% sequence identity to SEQ ID NO: 1 or SEQ ID NO: 5 is fused to the protein.

As used herein, the term "protein" relates to any polypeptide having an amino acid sequence, irrespective of the length or function of the polypeptide.

As used herein, the terms "expression of a protein" and "production of a protein" are used interchangeable and refer to the process of overexpressing a (heterologous) protein from DNA molecule such as a vector or plasmid or a chromosomal locus with the aim of harvesting and purifying said protein.

As used herein, the term "sequence identity" relates to the percentage of identity of two DNA, RNA or amino acid sequences obtained for a global alignment according to any alignment method known in the art.

In a preferred embodiment of the methods and cells of the invention, the non-toxic variant of diphtheria toxin has 80%, preferably 90%, even more preferred 95% sequence identity to SEQ ID NO: 2. In one embodiment, the non-toxic variant of diphtheria toxin has the sequence of SEQ ID NO: 2. SEQ ID NO: 2 is known as CRM197.

In the methods according to the invention, the protein is produced in a bacterial cell from the genus Corynebacterium, i.e., in a Corynebacterium cell. Use of a Corynebacterium cell is advantageous because diphtheria toxin is also expressed in a species of Corynebacterium. In addition, Corynebacterium cells are Gram-positive and thus do not produce lipopolysaccharides (LPS), which are toxic for humans. In a preferred embodiment, the Corynebacterium cell is non-pathogenic.

In a particularly preferred embodiment of the methods and cells of the invention, the Corynebacterium cell is a *Corynebacterium glutamicum* cell. C. *glutamicum* is known for its secretory production of proteins and does not have proteases in the supernatant, thus allowing for simple and straightforward purification of produced proteins. C. *glutamicum* has further been classified by the FDA as "generally recognized as safe" (GRAS) and "qualified presumption of safety" (QPS) by the EFSA, thereby omitting the risks associated with the use of pathogenic strains such as C. *diphtheriae.* As an established host for the production of heterologous proteins, C. *glutamicum* has been optimized for largest scale fermentation, including for the production of pharmaceutical products.

In a preferred embodiment, the Corynebacterium cell is from one of the following strains: C. *glutamicum* ATCC13032, ATCC 13058, ATCC 13175, ATCC 13744, ATCC 13745, ATCC 13806, ATCC 13869, ATCC 14017, ATCC 14020, ATCC 14067, ATCC 14068, ATCC 14747, ATCC 14751, ATCC 14752, ATCC 14915, ATCC 15243, ATCC 15354, ATCC 17965, ATCC 17966, ATCC 19223, ATCC 19240, ATCC 21645, ATCC 31808, ATCC 31830, ATCC 31832, ATCC 15990, ATCC 13825, LP6, WS 1863, 22243, DSM 20598 or a derivatives thereof. These strains have been metabolically engineered, mutagenized and/or evolved by serial passages in cultivation medium. As a result, these strains may contain one or more mutations, deletions, insertions, duplications or a combination of aforementioned modifications in comparison to wild-type strains. According to the invention, a signal peptide having at least 90% sequence identity to SEQ ID NO: 1 or SEQ ID NO: 5 is fused to the protein, i.e., the non-toxic variant of diphtheria toxin. In one embodiment, the signal peptide has at least 95% or most preferably 100% sequence identity to SEQ ID NO: 1 or SEQ ID NO: 5. In a particularly preferred embodiment, the signal peptide is SEQ ID NO: 1. SEQ ID NO: 1 is also known as signal peptide csn from *B. subtilis.* SEQ ID NO: 5 is also known as cspA from C. *ammoniagenes.*

The term "fused" as used herein means that the DNA sequence encoding the signal peptide is followed by the DNA sequence encoding the protein, so that upon expression, the two sequence are expressed as one contiguous polypeptide. In a preferred embodiment, the signal peptide is directly fused to the protein, i.e. there are no additional amino acids between the signal peptide and the protein. In this embodiment, after removal of the signal peptide, the sequence of the protein corresponds entirely to that of CRM197.

The inventors have surprisingly discovered that use of a signal peptide having at least 90% sequence identity to SEQ ID NO: 1 or SEQ ID NO: 5 greatly enhances secretion of the protein in Corynebacterium cells. In one embodiment, use of the signal peptide yields at least 150 mg/L of the protein.

In the methods according to the invention, the protein is produced using DNA molecules encoding the protein and the signal peptide. The cells used in the methods according to the invention may be transformed with these DNA molecules. As used herein, the terms "transform" or "transformed" refer to the application of any methods for introducing a foreign nucleic acid (such as a vector or plasmid) into a host cell with or without the presence of accompanying substances. Once introduced into the host cell, the nucleic acid may integrate into the genome or chromosome and become a fragment thereof, or remain as an extrachromosomal element, for the purpose of replication. Transformation of an appropriate host cell with, for example, an expression vector can be achieved using methods known in the art, such as electroporation.

In one embodiment, the protein is produced using a DNA molecule having at least 90%, preferably 95%, most preferably 100% sequence identity to SEQ ID NO: 3. In another embodiment, the cell of the invention comprises a DNA molecule having at least 90%, preferably 95%, most preferably 100% sequence identity to SEQ ID NO: 3. SEQ ID NO: 3 is a sequence that has been codon-optimized for expression in Corynebacterium cells, in particular C. *glutamicum.* As shown herein, use of DNA molecule having at least 90% sequence identity to SEQ ID NO: 3 leads to an improved expression and production of the protein.

The DNA molecule may be present in the Corynebacterium cell in the form of an extrachromosomal plasmid or at a chromosomal locus in one or multiple copies. According to the invention, DNA molecules may additionally comprise antibiotic resistance genes or other genetic markers to allow selection of successfully transformed cells.

In the methods according to the invention, the cells are cultured under conditions that allow expression of the protein. The skilled person is aware of how culture conditions can be adjusted to optimize protein expression. For example, culture temperature, culture duration and culture medium, as well as other culture conditions such as humidity, CO₂ content and agitation can be selected accordingly.

In one embodiment, the cell is first cultured before expression of the protein is induced. Protein expression may be rendered inducible by use of specific promoters that are sensitive to temperature or presence of particular compounds, such as IPTG, propionate, acetate, gluconate, maltose or arabinose. Examples of inducible promoters include P_{lacUV5}, P_{tac}, P_{trc}, P_{prpB}, P_{aceA}, P_{aceS}, P_{gntP}, P_{gntK}, P_{CJ1OX2}, P_{tac-M}, P_{malE1}, P_{glt1} and P_{BAD}.

In a preferred embodiment, the protein is secreted by the Corynebacterium cell. The inventors have found that using a signal peptide having at least 90% sequence identity to SEQ ID NO: 1 or SEQ ID NO: 5 facilitates secretion of the protein. In a particularly preferred embodiment, the protein is secreted into the extracytosolic space. During secretion, the signal peptide is removed from the protein, so that the final product does not comprise any additional amino acids compared to the sequence of CRM197 as depicted in SEQ ID NO: 2. According to the invention, the secreted protein has the correct 3D structure and folding, including two intramolecular disulfide bonds.

Once the protein has been expressed for an adequate amount of time, the protein can be harvested. Generally, the protein can be harvested and purified from the supernatant or cytosolic fraction. Harvesting may include additional steps to ensure purity of the protein fraction such as filtration, precipitation or chromatographic purification, e.g., immunoprecipitation, ammonium sulfate precipitation, ion exchange chromatography, size exclusion chromatography, immunochromatography or affinity chromatography. The skilled person knows how to obtain a secreted protein from the culture broth. In order to facilitate purification, the protein may be tagged.

In another aspect, the invention relates to a Corynebacterium cell capable of expressing a protein, wherein the protein is a non-toxic variant of diphtheria toxin and wherein a signal peptide having at least 90% sequence identity to SEQ ID NO: 1 or SEQ ID NO: 5 is fused to the protein. While the cells according to the invention express the non-toxic variant of diphtheria toxin to which a signal peptide having at least 90% sequence identity to SEQ ID NO: 1 or SEQ ID NO: 5, said signal peptide is cleaved from the non-toxic variant of diphtheria toxin during secretion, so that the cells according to the invention secrete a non-toxic variant of diphtheria toxin that does not contain any additional amino acids compared to SEQ ID NO: 2. In one embodiment, the cells are capable of expressing CRM 197.

As used herein, the term "a cell capable of expressing a protein" is understood to refer to a cell that contains the genetic material necessary to express the protein, e.g., a DNA or RNA molecule encoding the protein.

In a preferred embodiment, the methods for producing a non-toxic variant of diphtheria toxin according to the invention use these Corynebacterium cells.

In another aspect, the invention relates to methods for producing the cells capable of expressing a non-toxic variant of diphtheria toxin according to the invention. In one embodiment, a sensor is used to identify a Corynebacterium cell showing high expression of the non-toxic variant of diphtheria toxin.

In a preferred embodiment, the sensor is a fluorescent protein under the control of at least one heterologous promoter, which, in the wild type of the cell, controls the expression of a gene of which the expression in the wild-type cell depends on the amount of protein that is secreted across the cytoplasmic membrane into the extracytosolic space, so that the expression of the fluorescent protein depends on the amount of protein that is secreted across the cytoplasmic membrane into the extracytosolic space. Use of the sensor thus makes it possible to specifically select and propagate cells that show a high secretion of the protein into the extracytosolic space, by selecting cells showing a high fluorescence.

In yet another aspect, the invention relates to a DNA molecule encoding a non-toxic variant of diphtheria toxin, the DNA molecule comprising a part having at least 90% sequence identity to SEQ ID NO: 4. SEQ ID NO: 4 comprises the for C. *glutamicum* codon optimized sequence of CRM197 fused to signal peptide of SEQ ID NO: 1 under the control of the IPTG inducible promoter Ptac.

In yet another aspect, the invention relates to a DNA molecule encoding a non-toxic variant of diphtheria toxin, the DNA molecule comprising a part having at least 90% sequence identity to SEQ ID NO: 6. SEQ ID NO: 6 comprises the for C. *glutamicum* codon optimized sequence of CRM197 fused to signal peptide of SEQ ID NO: 5 under the control of the IPTG inducible promoter Ptac.

In a preferred embodiment, the DNA molecule is used in the methods for producing a non-toxic variant of diphtheria toxin according to the invention. In another preferred embodiment, the Corynebacterium cells capable of expressing a non-toxic variant of diphtheria toxin according to the invention are transformed with the DNA molecule according to the invention.

In one embodiment, the DNA molecule according to invention comprises a replicon for C. *glutamicum* such as pBL1, pCG1, pCGA1, pNG2, pEP2, pAJ228, pAM330, pHM1519, pCG100 or pSR1 and/or and a selectable marker, such as an antibiotic resistance gene conveying resistance to e.g, kanamycin, tetracyclin, spectinomycin or chloramphenicol.

The DNA molecule according to the invention additionally comprises a promoter. The promoter may be a constitutive promoter such as such as P_{*csp*B}, P*_{sod}*, *P_{aprE}*, P₁₈₀, P*_{dapA}*, P_{porB}, P*_{ilvC}*, a fully synthetic promoter such as P_{L10}, P_{L26}, P_{I16}, P_{I51}, P_{H30}, P_{H36} or an inducible promoter such as P_{lacUV5}, P_{tac}, P_{trc}, P_{prpB}, P_{aceA}, P_{aceB}, P_{gntP}, P_{gntK}, P_{CJ1OX2}, P_{tac-M}, P_{malE1}, P_{glt1} or P_{BAD}.

### Examples

### Example 1 - Codon optimzation

Three different codon optimized sequences were tested for CRM197 (codon_opt1 (SEQ ID NO: 3), codon_opt2 (SEQ ID NO: 7), codon_opt3 (SEQ ID NO: 8)) and compared to the wild type or native codon sequence (codonWT) found in *Corynebacterium diphtheriae.* The various codon optimized sequences were also fused to two different signal peptide (Sp) sequences (Sp1 and Sp2), which result in the secretion of CRM197 in C. *glutamicum.* A C. *glutamicum* strain was transformed with plasmids with various Sp and codon optimization constructs and cultivated for 24 hours in minimal medium CGXII supplemented with 4% glucose (Eggeling, L., and M. Bott. 2005. Handbook of Corynebacterium glutamicum, p. 535. CRC Press, Taylor Francis Group, Boca Raton, FL.). Supernatant samples were analyzed by Western Blot using Anti-Diphtheria Toxin Antibody (www.antibodies.com, Article No. A101706). The codon optimization that showed the highest signal under all conditions and combinations with signal peptides was codon opt 1 SEQ ID NO: 3 (Fig. 1 and 2).

### Example 2 - Selection of a signal peptide for secretion of CRM197 into the fermentation medium

A signal peptide library was prepared using the codon opt1 sequence of CRM197 SEQ ID NO: 3. With the signal peptide library 219 different signal peptides were tested for their secretion of CRM197 in C. *glutamicum.* Cultivation was done in CGXII supplemented with 4% glucose for 24 hours and supernatant was analyzed by Western Blot using Anti-Diphtheria Toxin Antibody. Cultures were induced with 1 mM IPTG. The signal peptide resulting in the highest secretion of CRM197 in C. *glutamicum* was identified as the signal peptide sequence of the csn protein of B. *subtilis* SEQ ID NO: 1 (Fig. 3).

Various signal peptides are known in the art that have previously resulted in good protein secretion in C. *glutamicum* (Freudl R. Beyond amino acids: Use of the Corynebacterium glutamicum cell factory for the secretion of heterologous proteins. J Biotechnol. 2017 Sep 20;258:101-109). Five of these were selected (Sp_cg1514, Sp_nprE, Sp_cspA, Sp_CgR0949 and Sp_phoD) to compare to the signal peptide hit from the library. The signal peptides selected were also in the library and this experiment was done to further support the result of the signal peptide library screening. Again cultivation was done in CGXII supplemented with 4% glucose for 24 hours and protein expression was induced with 1 mM IPTG.

In Fig. 4, the western blot (WB) using Anti-Diphtheria Toxin Antibody to visualize the CRM197 protein from the various culture supernatants clearly shows that the combination of CRM197 SEQ ID NO: 3 and csn signal peptide SEQ ID NO: 1 (lane 1) is superior to the other signal peptide combinations with CRM197. The only other signal peptide that shows a clear production of full length CRM197 is the *cspA* signal peptide in combination with CRM197 (lane 4).

### Example 3 - Construction of functional expression cassettes

A C. *glutamicum* strain ATCC13032 expressing codon opt 1 CRM197 SEQ ID NO: 3 fused to csn signal peptide SEQ ID NO: 1 from B. *subtilis* was cultured under fed batch conditions (1 L DasGip vessel). The CRM197 signal peptide construct is under the control of the inducible Ptac promoter and expressed from a vector containing the pBL1 origin of replication for C. *glutamicum* and the laclq gene for insuring tight expression control SEQ ID NO: 4. In the batch phase with 3% glucose the focus was on preparing biomass, while in the fedbatch phase, when up to 12% final glucose is fed, induction with IPTG takes place and CRM197 production starts. Production of up to 150 mg/L CRM197 in supernatant was found and the protein showed high stability in the fermentation broth. Seven hours after feed was ended, no significant degradation of protein was observed (Fig. 5).
SEQ ID NO: 1 - csn 
atgaaaatca gtatgcaaaa agcagatttt tggaaaaaag cagcgatctc attacttgtt
ttcaccatgt tttttaccct gatgatgagc gaaacggttt ttgcg
SEQ ID NO: 2 - CRM197 
GADDVVDSSKSFVMENFSSYHGTKPGYVDSIQKGIQKPKSGTQGNYDDDWKEFYSTDNKYDAAGYSVDNEN
PLSGKAGGVVKVTYPGLTKVLALKVDNAETIKKELGLSLTEPLMEQVGTEEFIKRFGDGASRVVLSLPFAEGSS
SVEYINNWEQAKALSVELEINFETRGKRGQDAMYEYMAQACAGNRVRRSVGSSLSCINLDWDVIRDKTKTKIE
SLKEHGPIKNKMSESPNKTVSEEKAKQYLEEFHQTALEHPELSELKTVTGTNPVFAGANYAAWAVNVAQVIDS
ETADNLEKTTAALSILPGIGSVMGIADGAVHHNTEEIVAQSIALSSLMVAQAIPLVGELVDIGFAAYNFVESIINLF
QVVHNSYNRPAYSPGHKTQPFLHDGYAVSWNTVEDSIIRTGFQGESGHDIKITAENTPLPIAGVLLPTIPGKLD
VNKSKTHISVNGRKIRMRCRAIDGDVTFCRPKSPVYVGNGVHANLHVAFHRSSSEKIHSNEISSDSIGVLGYQK
TVDHTKVNSKLSLFFEIKS
SEQ ID NO: 3 - CRM197 codon opt1 
ggcgctgacgacgttgttgactcctccaagtccttcgttatggagaacttctcctcctaccacggcaccaagccaggctacgttgactccatccagaagggc
atccagaagccaaagtccggcacccagggcaactacgacgacgactggaaggagttctactccaccgacaacaagtacgacgctgctggctactccgt
tgacaacgagaacccactgtccggcaaggctggcggcgttgttaaggttacctacccaggcctgaccaaggttctggctctgaaggttgacaacgctgag
accatcaagaaggagctgggcctgtccctgaccgagccactgatggagcaggttggcaccgaggagttcatcaagcgcttcggcgacggcgcttcccg
cgttgttctgtccctgccattcgctgagggctcctcctccgttgagtacatcaacaactgggagcaggctaaggctctgtccgttgagctggagatcaacttcg
agacccgcggcaagcgcggccaggacgctatgtacgagtacatggctcaggcttgcgctggcaaccgcgttcgccgctccgttggctcctccctgtcctgc
atcaacctggactgggacgttatccgcgacaagaccaagaccaagatcgagtccctgaaggagcacggcccaatcaagaacaagatgtccgagtccc
caaacaagaccgtttccgaggagaaggctaagcagtacctggaggagttccaccagaccgctctggagcacccagagctgtccgagctgaagaccgtt
 
accggcaccaacccagttttcgctggcgctaactacgctgcttgggctgttaacgttgctcaggttatcgactccgagaccgctgacaacctggagaagacc
accgctgctctgtccatcctgccaggcatcggctccgttatgggcatcgctgacggcgctgttcaccacaacaccgaggagatcgttgctcagtccatcgctc
tgtcctccctgatggttgctcaggctatcccactggttggcgagctggttgacatcggcttcgctgcttacaacttcgttgagtccatcatcaacctgttccaggtt
gttcacaactcctacaaccgcccagcttactccccaggccacaagacccagccattcctgcacgacgggtacgctgtcagctggaacaccgttgaggact
ccatcatccgcaccggcttccagggcgagtccggccacgacatcaagatcaccgctgagaacaccccactgccaatcgctggcgttctgctgccaacca
tcccaggcaagctggacgttaacaagtccaagacccacatctccgttaacggccgcaagatccgcatgcgctgccgcgctatcgacggcgacgttacctt
ctgccgcccaaagtccccagtttacgttggcaacggcgttcacgctaacctgcacgttgctttccaccgctcctcctccgagaagatccactccaacgagat
ctcctccgactccatcggcgttctgggctaccagaagaccgttgaccacaccaaggttaactccaagctgtccctgttcttcgagatcaagtcc
SEQ ID NO: 4 - PTAC_csn_Opt1 
ATGAAAATCAGTATGCAAAAAGCAGATTTTTGGAAAAAAGCAGCGATCTC
ATTACTTGTTTTCACCATGTTTTTTACCCTGATGATGAGCGAAACGGTTT
TTGCGGGCGCTGACGACGTTGTTGACTCCTCCAAGTCCTTCGTTATGGAG
AACTTCTCCTCCTACCACGGCACCAAGCCAGGCTACGTTGACTCCATCCA
GAAGGGCATCCAGAAGCCAAAGTCCGGCACCCAGGGCAACTACGACGACG
ACTGGAAGGAGTTCTACTCCACCGACAACAAGTACGACGCTGCTGGCTAC
TCCGTTGACAACGAGAACCCACTGTCCGGCAAGGCTGGCGGCGTTGTTAA
GGTTACCTACCCAGGCCTGACCAAGGTTCTGGCTCTGAAGGTTGACAACG
CTGAGACCATCAAGAAGGAGCTGGGCCTGTCCCTGACCGAGCCACTGATG
GAGCAGGTTGGCACCGAGGAGTTCATCAAGCGCTTCGGCGACGGCGCTTC
CCGCGTTGTTCTGTCCCTGCCATTCGCTGAGGGCTCCTCCTCCGTTGAGT
ACATCAACAACTGGGAGCAGGCTAAGGCTCTGTCCGTTGAGCTGGAGATC
AACTTCGAGACCCGCGGCAAGCGCGGCCAGGACGCTATGTACGAGTACAT
GGCTCAGGCTTGCGCTGGCAACCGCGTTCGCCGCTCCGTTGGCTCCTCCC
TGTCCTGCATCAACCTGGACTGGGACGTTATCCGCGACAAGACCAAGACC
AAGATCGAGTCCCTGAAGGAGCACGGCCCAATCAAGAACAAGATGTCCGA
GTCCCCAAACAAGACCGTTTCCGAGGAGAAGGCTAAGCAGTACCTGGAGG
AGTTCCACCAGACCGCTCTGGAGCACCCAGAGCTGTCCGAGCTGAAGACC
GTTACCGGCACCAACCCAGTTTTCGCTGGCGCTAACTACGCTGCTTGGGC
TGTTAACGTTGCTCAGGTTATCGACTCCGAGACCGCTGACAACCTGGAGA
AGACCACCGCTGCTCTGTCCATCCTGCCAGGCATCGGCTCCGTTATGGGC
ATCGCTGACGGCGCTGTTCACCACAACACCGAGGAGATCGTTGCTCAGTC
CATCGCTCTGTCCTCCCTGATGGTTGCTCAGGCTATCCCACTGGTTGGCG
AGCTGGTTGACATCGGCTTCGCTGCTTACAACTTCGTTGAGTCCATCATC
AACCTGTTCCAGGTTGTTCACAACTCCTACAACCGCCCAGCTTACTCCCC
AGGCCACAAGACCCAGCCATTCCTGCACGACGGGTACGCTGTCAGCTGGA
ACACCGTTGAGGACTCCATCATCCGCACCGGCTTCCAGGGCGAGTCCGGC
CACGACATCAAGATCACCGCTGAGAACACCCCACTGCCAATCGCTGGCGT
TCTGCTGCCAACCATCCCAGGCAAGCTGGACGTTAACAAGTCCAAGACCC
ACATCTCCGTTAACGGCCGCAAGATCCGCATGCGCTGCCGCGCTATCGAC
GGCGACGTTACCTTCTGCCGCCCAAAGTCCCCAGTTTACGTTGGCAACGG
CGTTCACGCTAACCTGCACGTTGCTTTCCACCGCTCCTCCTCCGAGAAGA
TCCACTCCAACGAGATCTCCTCCGACTCCATCGGCGTTCTGGGCTACCAG
AAGACCGTTGACCACACCAAGGTTAACTCCAAGCTGTCCCTGTTCTTCGA
GATCAAGTCCTAAGGATCCCCGGGTACCGAGCTCGAATACACTGGCCGTC
GTTTTACAGCCAAGCTTGGCTGTTTTGGCGGATGAGAGAAGATTTTCAGC
CTGATACAGATTAAATCAGAACGCAGAAGCGGTCTGATAAAACAGAATTT
GCCTGGCGGCAGTAGCGCGGTGGTCCCACCTGACCCCATGCCGAACTCAG
AAGTGAAACGCCGTAGCGCCGATGGTAGTGTGGGGTCTCCCCATGCGAGA
GTAGGGAACTGCCAGGCATCAAATAAAACGAAAGGCTCAGTCGAAAGACT
GGGCCTTTCGTTTTATCTGTTGTTTGTCGGTGAACGCTCTCCTGAGTAGG
ACAAATCCGCCGGGAGCGGATTTGAACGTTGCGAAGCAACGGCCCGGAGG
GTGGCGGGCAGGACGCCCGCCATAAACTGCCAGGCATCAAATTAAGCAGA
AGGCCATCCTGACGGATGGCCTTTTTGCGTTTCTACAAACTCTTTTGTTT
ATTTTTCTAAATACATTCAAATATGTATCCGCTCATGAGACAATAACCCT
GATAAATGCTTCAATAATATTGAAAAAGGAAGAGTATGAGTATTCAACAT
TTCCGTGTCGCCCTTATTCCCTTTTTTGCGGCATTTTGCCTTCCTGTTTT
TGCTCACCCAGAAACGCTGGTGAAAGTAAAAGATGCTGAAGATCAGTTGG
 
GTGCACGAGTGGGTTACATCGAACTGGATCTCAACAGCGGTAAGATCCTT
GAGAGTTTTCGCCCCGAAGAACGTTTTCCAATGATGAGCACTTTTAAAGT
TCTGCTATGTGGCGCGGTATTATCCCGTGTTGACGCCGGGCAAGAGCAAC
TCGGTCGCCGCATACACTATTCTCAGAATGACTTGGTTGAGTAATTCGTA
ATCATGTCATAGGCTCTTCCGCTTCCTCGCTCACTGACTCGCTGCGCTCG
GTCGTTCGGCTGCGGCGAGCGGTATCAGCTCACTCAAAGGCGGTAATACG
GTTATCCACAGAATCAGGGGATAACGCAGGAAAGAACATGTGAGCAAAAG
GCCAGCAAAAGGCCAGGAACCGTAAAAAGGCCGCGTTGCTGGCGTTTTTC
CATAGGCTCCGCCCCCCTGACGAGCATCACAAAAATCGACGCTCAAGTCA
GAGGTGGCGAAACCCGACAGGACTATAAAGATACCAGGCGTTTCCCCCTG
GAAGCTCCCTCGTGCGCTCTCCTGTTCCGACCCTGCCGCTTACCGGATAC
CTGTCCGCCTTTCTCCCTTCGGGAAGCGTGGCGCTTTCTCATAGCTCACG
CTGTAGGTATCTCAGTTCGGTGTAGGTCGTTCGCTCCAAGCTGGGCTGTG
TGCACGAACCCCCCGTTCAGCCCGACCGCTGCGCCTTATCCGGTAACTAT
CGTCTTGAGTCCAACCCGGTAAGACACGACTTATCGCCACTGGCAGCAGC
CACTGGTAACAGGATTAGCAGAGCGAGGTATGTAGGCGGTGCTACAGAGT
TCTTGAAGTGGTGGCCTAACTACGGCTACACTAGAAGAACAGTATTTGGT
ATCTGCGCTCTGCTGAAGCCAGTTACCTTCGGAAAAAGAGTTGGTAGCTC
TTGATCCGGCAAACAAACCACCGCTGGTAGCGGTGGTTTTTTTGTTTGCA
AGCAGCAGATTACGCGCAGAAAAAAAGGATCTCAAGAAGATCCTTTGATC
TTTTCTACGGGGTCTGACGCTCAGTGGAACGAAAACTCACGTTAAGGGAT
TTTGGTCATGAGATTATCAAAAAGGATCTTCACCTAGATCCTTTTGGGGG
GGGGGGGAAAGCCACGTTGTGTCTCAAAATCTCTGATGTTACATTGCACA
AGATAAAAATATATCATCATGAACAATAAAACTGTCTGCTTACATAAACA
GTAATACAAGGGGTGTTATGAGCCATATTCAACGGGAAACGTCTTGCTCG
AGGCCGCGATTAAATTCCAACATGGATGCTGATTTATATGGGTATAAATG
GGCTCGCGATAATGTCGGGCAATCAGGTGCGACAATCTATCGATTGTATG
GGAAGCCCGATGCGCCAGAGTTGTTTCTGAAACATGGCAAAGGTAGCGTT
GCCAATGATGTTACAGATGAGATGGTCAGACTAAACTGGCTGACGGAATT
TATGCCTCTTCCGACCATCAAGCATTTTATCCGTACTCCTGATGATGCAT
GGTTACTCACCACTGCGATCCCCGGGAAAACAGCATTCCAGGTATTAGAA
GAATATCCTGATTCAGGTGAAAATATTGTTGATGCGCTGGCAGTGTTCCT
GCGCCGGTTGCATTCGATTCCTGTTTGTAATTGTCCTTTTAACAGCGATC
GCGTATTTCGTCTCGCTCAGGCGCAATCACGAATGAATAACGGTTTGGTT
GATGCGAGTGATTTTGATGACGAGCGTAATGGCTGGCCTGTTGAACAAGT
CTGGAAAGAAATGCATAAGCTTTTGCCATTCTCACCGGATTCAGTCGTCA
CTCATGGTGATTTCTCACTTGATAACCTTATTTTTGACGAGGGGAAATTA
ATAGGTTGTATTGATGTTGGACGAGTCGGAATCGCAGACCGATACCAGGA
TCTTGCCATCCTATGGAACTGCCTCGGTGAGTTTTCTCCTTCATTACAGA
AACGGCTTTTTCAAAAATATGGTATTGATAATCCTGATATGAATAAATTG
CAGTTTCATTTGATGCTCGATGAGTTTTTCTAATCAGAATTGGTTAATTG
GTTGTAACACTGGCAGAGCATTACGCTGACTTGACGGGACGGCGGCTTTG
TTGAATAAATCGAACTTTTGCTGAGTTGAAGGATCAGATCACGCATCTTC
CCGACAACGCAGACCGTTCCGTGGCAAAGCAAAAGTTCAAAATCACCAAC
TGGTCCACCTACAACAAAGCTCTCATCAACCGTGGCTCCCTCACTTTCTG
GCTGGATGATGGGGCGATTCAGGCCTGGTATGAGTCAGCAACACCTTCTT
CACGAGGCAGACCTCAGCGCCCCCCCCCCCCTAGCTTGTCTACGTCTGAT
GCTTTGAATCGGACGGACTTGCCGATCTTGTATGCGGTGATTTTTCCCTC
GTTTGCCCACTTTTTAATGGTGGCCGGGGTGAGAGCTACGCGGGCGGCGA
CCTGCTGCGCTGTGATCCAATATTCGGGGTCGTTCACTGGTTCCCCTTTC
TGATTTCTGGCATAGAAGAACCCCCGTGAACTGTGTGGTTCCGGGGGTTG
CTGATTTTTGCGAGACTTCTCGCGCAATTCCCTAGCTTAGGTGAAAACAC
CATGAAACACTAGGGAAACACCCATGAAACACCCATTAGGGCAGTAGGGC
GGCTTCTTCGTCTAGGGCTTGCATTTGGGCGGTGATCTGGTCTTTAGCGT
GTGAAAGTGTGTCGTAGGTGGCGTGCTCAATGCACTCGAACGTCACGTCA
TTTACCGGGTCACGGTGGGCAAAGAGAACTAGTGGGTTAGACATTGTTTT
CCTCGTTGTCGGTGGTGGTGAGCTTTTCTAGCCGCTCGGTAAACGCGGCG
ATCATGAACTCTTGGAGGTTTTCACCGTTCTGCATGCCTGCGCGCTTCAT
GTCCTCACGTAGTGCCAAAGGAACGCGTGCGGTGACCACGACGGGCTTAG
CCTTTGCCTGCGCTTCTAGTGCTTCGATGGTGGCTTGTGCCTGCGCTTGC
TGCGCCTGTAGTGCCTGTTGAGCTTCTTGTAGTTGCTGTTCTAGCTGTGC
CTTGGTTGCCATGCTTTAAGACTCTAGTAGCTTTCCTGCGATATGTCATG
CGCATGCGTAGCAAACATTGTCCTGCAACTCATTCATTATGTGCAGTGCT
CCTGTTACTAGTCGTACATACTCATATTTACCTAGTCTGCATGCAGTGCA
TGCACATGCAGTCATGTCGTGCTAATGTGTAAAACATGTACATGCAGATT
 
GCTGGGGGTGCAGGGGGCGGAGCCACCCTGTCCATGCGGGGTGTGGGGCT
TGCCCCGCCGGTACAGACAGTGAGCACCGGGGCACCTAGTCGCGGATACC
CCCCCTAGGTATCGGACACGTAACCCTCCCATGTCGATGCAAATCTTTAA
CATTGAGTACGGGTAAGCTGGCACGCATAGCCAAGCTAGGCGGCCACCAA
ACACCACTAAAAATTAATAGTTCCTAGACAAGACAAACCCCCGTGCGAGC
TACCAACTCATATGCACGGGGGCCACATAACCCGAAGGGGTTTCAATTGA
CAACCATAGCACTAGCTAAGACAACGGGCACAACACCCGCACAAACTCGC
ACTGCGCAACCCCGCACAACATCGGGTCTAGGTAACACTGAAATAGAAGT
GAACACCTCTAAGGAACCGCAGGTCAATGAGGGTTCTAAGGTCACTCGCG
CTAGGGCGTGGCGTAGGCAAAACGTCATGTACAAGATCACCAATAGTAAG
GCTCTGGCGGGGTGCCATAGGTGGCGCAGGGACGAAGCTGTTGCGGTGTC
CTGGTCGTCTAACGGTGCTTCGCAGTTTGAGGGTCTGCAAAACTCTCACT
CTCGCTGGGGGTCACCTCTGGCTGAATTGGAAGTCATGGGCGAACGCCGC
ATTGAGCTGGCTATTGCTACTAAGAATCACTTGGCGGCGGGTGGCGCGCT
CATGATGTTTGTGGGCACTGTTCGACACAACCGCTCACAGTCATTTGCGC
AGGTTGAAGCGGGTATTAAGACTGCGTACTCTTCGATGGTGAAAACATCT
CAGTGGAAGAAAGAACGTGCACGGTACGGGGTGGAGCACACCTATAGTGA
CTATGAGGTCACAGACTCTTGGGCGAACGGTTGGCACTTGCACCGCAACA
TGCTGTTGTTCTTGGATCGTCCACTGTCTGACGATGAACTCAAGGCGTTT
GAGGATTCCATGTTTTCCCGCTGGTCTGCTGGTGTGGTTAAGGCCGGTAT
GGACGCGCCACTGCGTGAGCACGGGGTCAAACTTGATCAGGTGTCTACCT
GGGGTGGAGACGCTGCGAAAATGGCAACCTACCTCGCTAAGGGCATGTCT
CAGGAACTGACTGGCTCCGCTACTAAAACCGCGTCTAAGGGGTCGTACAC
GCCGTTTCAGATGTTGGATATGTTGGCCGATCAAAGCGACGCCGGCGAGG
ATATGGACGCTGTTTTGGTGGCTCGGTGGCGTGAGTATGAGGTTGGTTCT
AAAAACCTGCGTTCGTCCTGGTCACGTGGGGCTAAGCGTGCTTTGGGCAT
TGATTACATAGACGCTGATGTACGTCGTGAAATGGAAGAAGAACTGTACA
AGCTCGCCGGTCTGGAAGCACCGGAACGGGTCGAATCAACCCGCGTTGCT
GTTGCTTTGGTGAAGCCCGATGATTGGAAACTGATTCAGTCTGATTTCGC
GGTTAGGCAGTACGTTCTAGATTGCGTGGATAAGGCTAAGGACGTGGCCG
CTGCGCAACGTGTCGCTAATGAGGTGCTGGCAAGTCTGGGTGTGGATTCC
ACCCCGTGCATGATCGTTATGGATGATGTGGACTTGGACGCGGTTCTGCC
TACTCATGGGGACGCTACTAAGCGTGATCTGAATGCGGCGGTGTTCGCGG
GTAATGAGCAGACTATTCTTCGCACCCACTAAAAGCGGCATAAACCCCGT
TCGATATTTTGTGCGATGAATTTATGGTCAATGTCGCGGGGGCAAACTAT
GATGGGTCTTGTTGTTGACAATGGCTGATTTCATCAGGAATGGAACTGTC
ATGCTGTTATGTGCCTGGCTCCTAATCAAAGCTGGGGACAATGGGTTGCC
CCGTTGATCTGATCTAGTTCGGATTGGCGGGGCTTCACTGTATCTGGGGG
TGGCATCGTGAATAGATTGCACACCGTAGTGGGCAGTGTGCACACCATAG
TGGCCATGAGCACCACCACCCCCAGGGACGCCGACGGCGCGAAGCTCTGC
GCCTGGTGCGGCTCGGAGATCAAGCAATCCGGCGTCGGCCGGAGCCGGGA
CTACTGCCGCCGCTCCTGCCGCCAGCGGGCGTACGAGGCCCGGCGCCAGC
GCGAGGCGATCGTGTCCGCCGTGGCGTCGGCAGTCGCTCGCCGAGATACG
TCACGTGACGAAATGCAGCAGCCTTCCATTCCGTCACGTGACGAAACTCG
GGCCGCAGGTCAGAGCACGGTTCCGCCCGCTCCGGCCCTGCCGGACCCCC
GGCATCCCGCAAGAGGCCCGGCAGTACCGGCATAACCAAGCCTATGCCTA
CAGCATCCAGGGTGACGGTGCCGAGGATGACGATGAGCGCATTGTTAGAT
TTCATACACGGTGCCTGACTGCGTTAGCAATTTAACTGTGATAAACTACC
GCATTAAAGCTTATCGATGATAAGCTGTCAAACATGGCCTGTCGCTTGCG
GTATTCGGAATCTTGCACGCCCTCGCTCACTGCCCGCTTTCCAGTCGGGA
AACCTGTCGTGCCAGCTGCATTAATGAATCGGCCAACGCGCGGGGAGAGG
CGGTTTGCGTATTGGGCGCCAGGGTGGTTTTTCTTTTCACCAGTGAGACG
GGCAACAGCTGATTGCCCTTCACCGCCTGGCCCTGAGAGAGTTGCAGCAA
GCGGTCCACGCTGGTTTGCCCCAGCAGGCGAAAATCCTGTTTGATGGTGG
TTAACGGCGGGATATAACATGAGCTGTCTTCGGTATCGTCGTATCCCACT
ACCGAGATATCCGCACCAACGCGCAGCCCGGACTCGGTAATGGCGCGCAT
TGCGCCCAGCGCCATCTGATCGTTGGCAACCAGCATCGCAGTGGGAACGA
TGCCCTCATTCAGCATTTGCATGGTTTGTTGAAAACCGGACATGGCACTC
CAGTCGCCTTCCCGTTCCGCTATCGGCTGAATTTGATTGCGAGTGAGATA
TTTATGCCAGCCAGCCAGACGCAGACGCGCCGAGACAGAACTTAATGGGC
CCGCTAACAGCGCGATTTGCTGGTGACCCAATGCGACCAGATGCTCCACG
CCCAGTCGCGTACCGTCTTCATGGGAGAAAATAATACTGTTGATGGGTGT
CTGGTCAGAGACATCAAGAAATAACGCCGGAACATTAGTGCAGGCAGCTT
CCACAGCAATGGCATCCTGGTCATCCAGCGGATAGTTAATGATCAGCCCA
CTGACGCGTTGCGCGAGAAGATTGTGCACCGCCGCTTTACAGGCTTCGAC
 
GCCGCTTCGTTCTACCATCGACACCACCACGCTGGCACCCAGTTGATCGG
CGCGAGATTTAATCGCCGCGACAATTTGCGACGGCGCGTGCAGGGCCAGA
CTGGAGGTGGCAACGCCAATCAGCAACGACTGTTTGCCCGCCAGTTGTTG
TGCCACGCGGTTGGGAATGTAATTCAGCTCCGCCATCGCCGCTTCCACTT
TTTCCCGCGTTTTCGCAGAAACGTGGCTGGCCTGGTTCACCACGCGGGAA
ACGGTCTGATAAGAGACACCGGCATACTCTGCGACATCGTATAACGTTAC
TGGTTTCACATTCACCACCCTGAATTGACTCTCTTCCGGGCGCTATCATG
CCATACCGCGAAAGGTTTTGCACCATTCGATGGTGTCAACGTAAATGCAT
GCCGCTTCGCCTTCGCGCGCGAATTGCAAGCTGATCCGGGCTTATCGACT
GCACGGTGCACCAATGCTTCTGGCGTCAGGCAGCCATCGGAAGCTGTGGT
ATGGCTGTGCAGGTCGTAAATCACTGCATAATTCGTGTCGCTCAAGGCGC
ACTCCCGTTCTGGATAATGTTTTTTGCGCCGACATCATAACGGTTCTGGC
AAATATTCTGAAATGAGCTGTTGACAATTAATCATCGGCTCGTATAATGT
GTGGAATTGTGAGCGGATAACAATTTCACACAGGAAACAGAATTAAAAGA
TATGACCATGATTACGCCAAGCTTGCATGCCTGCAGGTCGAC
SEQ ID NO: 5 - cspA
   atgaaacgcatgaaatcgctggctgcggcgctcaccgtcgctggggccatgctggccgcacctgtggcaacggca
SEQ ID NO: 6 - PTAC_cspA_Opt1 
atgaaacgcatgaaatcgctggctgcggcgctcaccgtcgctggggccatgctggccgcacctgtggcaacggcaggcgctgacgacgttgttgactcct
ccaagtccttcgttatggagaacttctcctcctaccacggcaccaagccaggctacgttgactccatccagaagggcatccagaagccaaagtccggcac
ccagggcaactacgacgacgactggaaggagttctactccaccgacaacaagtacgacgctgctggctactccgttgacaacgagaacccactgtccg
gcaaggctggcggcgttgttaaggttacctacccaggcctgaccaaggttctggctctgaaggttgacaacgctgagaccatcaagaaggagctgggcct
gtccctgaccgagccactgatggagcaggttggcaccgaggagttcatcaagcgcttcggcgacggcgcttcccgcgttgttctgtccctgccattcgctga
gggctcctcctccgttgagtacatcaacaactgggagcaggctaaggctctgtccgttgagctggagatcaacttcgagacccgcggcaagcgcggcca
ggacgctatgtacgagtacatggctcaggcttgcgctggcaaccgcgttcgccgctccgttggctcctccctgtcctgcatcaacctggactgggacgttatc
cgcgacaagaccaagaccaagatcgagtccctgaaggagcacggcccaatcaagaacaagatgtccgagtccccaaacaagaccgtttccgagga
gaaggctaagcagtacctggaggagttccaccagaccgctctggagcacccagagctgtccgagctgaagaccgttaccggcaccaacccagttttcgc
tggcgctaactacgctgcttgggctgttaacgttgctcaggttatcgactccgagaccgctgacaacctggagaagaccaccgctgctctgtccatcctgcca
ggcatcggctccgttatgggcatcgctgacggcgctgttcaccacaacaccgaggagatcgttgctcagtccatcgctctgtcctccctgatggttgctcagg
ctatcccactggttggcgagctggttgacatcggcttcgctgcttacaacttcgttgagtccatcatcaacctgttccaggttgttcacaactcctacaaccgccc
agcttactccccaggccacaagacccagccattcctgcacgacgggtacgctgtcagctggaacaccgttgaggactccatcatccgcaccggcttccag
ggcgagtccggccacgacatcaagatcaccgctgagaacaccccactgccaatcgctggcgttctgctgccaaccatcccaggcaagctggacgttaa
caagtccaagacccacatctccgttaacggccgcaagatccgcatgcgctgccgcgctatcgacggcgacgttaccttctgccgcccaaagtccccagttt
acgttggcaacggcgttcacgctaacctgcacgttgctttccaccgctcctcctccgagaagatccactccaacgagatctcctccgactccatcggcgttct
gggctaccagaagaccgttgaccacaccaaggttaactccaagctgtccctgttcttcgagatcaagtcctaaggatccccgggtaccgagctcgaatac
actggccgtcgttttacagccaagcttggctgttttggcggatgagagaagattttcagcctgatacagattaaatcagaacgcagaagcggtctgataaaa
cagaatttgcctggcggcagtagcgcggtggtcccacctgaccccatgccgaactcagaagtgaaacgccgtagcgccgatggtagtgtggggtctccc
catgcgagagtagggaactgccaggcatcaaataaaacgaaaggctcagtcgaaagactgggcctttcgttttatctgttgtttgtcggtgaacgctctcctg
agtaggacaaatccgccgggagcggatttgaacgttgcgaagcaacggcccggagggtggcgggcaggacgcccgccataaactgccaggcatca
aattaagcagaaggccatcctgacggatggcctttttgcgtttctacaaactcttttgtttatttttctaaatacattcaaatatgtatccgctcatgagacaataacc
ctgataaatgcttcaataatattgaaaaaggaagagtatgagtattcaacatttccgtgtcgcccttattcccttttttgcggcattttgccttcctgtttttgctcaccc
agaaacgctggtgaaagtaaaagatgctgaagatcagttgggtgcacgagtgggttacatcgaactggatctcaacagcggtaagatccttgagagttttc
gccccgaagaacgttttccaatgatgagcacttttaaagttctgctatgtggcgcggtattatcccgtgttgacgccgggcaagagcaactcggtcgccgcat
acactattctcagaatgacttggttgagtaattcgtaatcatgtcataggctcttccgcttcctcgctcactgactcgctgcgctcggtcgttcggctgcggcgag
cggtatcagctcactcaaaggcggtaatacggttatccacagaatcaggggataacgcaggaaagaacatgtgagcaaaaggccagcaaaaggcca
ggaaccgtaaaaaggccgcgttgctggcgtttttccataggctccgcccccctgacgagcatcacaaaaatcgacgctcaagtcagaggtggcgaaacc
cgacaggactataaagataccaggcgtttccccctggaagctccctcgtgcgctctcctgttccgaccctgccgcttaccggatacctgtccgcctttctccctt
cgggaagcgtggcgctttctcatagctcacgctgtaggtatctcagttcggtgtaggtcgttcgctccaagctgggctgtgtgcacgaaccccccgttcagccc
gaccgctgcgccttatccggtaactatcgtcttgagtccaacccggtaagacacgacttatcgccactggcagcagccactggtaacaggattagcagag
cgaggtatgtaggcggtgctacagagttcttgaagtggtggcctaactacggctacactagaagaacagtatttggtatctgcgctctgctgaagccagttac
 
cttcggaaaaagagttggtagctcttgatccggcaaacaaaccaccgctggtagcggtggtttttttgtttgcaagcagcagattacgcgcagaaaaaaag
gatctcaagaagatcctttgatcttttctacggggtctgacgctcagtggaacgaaaactcacgttaagggattttggtcatgagattatcaaaaaggatcttca
cctagatccttttggggggggggggaaagccacgttgtgtctcaaaatctctgatgttacattgcacaagataaaaatatatcatcatgaacaataaaactgt
ctgcttacataaacagtaatacaaggggtgttatgagccatattcaacgggaaacgtcttgctcgaggccgcgattaaattccaacatggatgctgatttatat
gggtataaatgggctcgcgataatgtcgggcaatcaggtgcgacaatctatcgattgtatgggaagcccgatgcgccagagttgtttctgaaacatggcaa
aggtagcgttgccaatgatgttacagatgagatggtcagactaaactggctgacggaatttatgcctcttccgaccatcaagcattttatccgtactcctgatga
tgcatggttactcaccactgcgatccccgggaaaacagcattccaggtattagaagaatatcctgattcaggtgaaaatattgttgatgcgctggcagtgttcc
tgcgccggttgcattcgattcctgtttgtaattgtccttttaacagcgatcgcgtatttcgtctcgctcaggcgcaatcacgaatgaataacggtttggttgatgcga
gtgattttgatgacgagcgtaatggctggcctgttgaacaagtctggaaagaaatgcataagcttttgccattctcaccggattcagtcgtcactcatggtgattt
ctcacttgataaccttatttttgacgaggggaaattaataggttgtattgatgttggacgagtcggaatcgcagaccgataccaggatcttgccatcctatggaa
ctgcctcggtgagttttctccttcattacagaaacggctttttcaaaaatatggtattgataatcctgatatgaataaattgcagtttcatttgatgctcgatgagttttt
ctaatcagaattggttaattggttgtaacactggcagagcattacgctgacttgacgggacggcggctttgttgaataaatcgaacttttgctgagttgaaggat
cagatcacgcatcttcccgacaacgcagaccgttccgtggcaaagcaaaagttcaaaatcaccaactggtccacctacaacaaagctctcatcaaccgt
ggctccctcactttctggctggatgatggggcgattcaggcctggtatgagtcagcaacaccttcttcacgaggcagacctcagcgcccccccccccctagc
ttgtctacgtctgatgctttgaatcggacggacttgccgatcttgtatgcggtgatttttccctcgtttgcccactttttaatggtggccggggtgagagctacgcgg
gcggcgacctgctgcgctgtgatccaatattcggggtcgttcactggttcccctttctgatttctggcatagaagaacccccgtgaactgtgtggttccgggggtt
gctgatttttgcgagacttctcgcgcaattccctagcttaggtgaaaacaccatgaaacactagggaaacacccatgaaacacccattagggcagtaggg
cggcttcttcgtctagggcttgcatttgggcggtgatctggtctttagcgtgtgaaagtgtgtcgtaggtggcgtgctcaatgcactcgaacgtcacgtcatttacc
gggtcacggtgggcaaagagaactagtgggttagacattgttttcctcgttgtcggtggtggtgagcttttctagccgctcggtaaacgcggcgatcatgaact
cttggaggttttcaccgttctgcatgcctgcgcgcttcatgtcctcacgtagtgccaaaggaacgcgtgcggtgaccacgacgggcttagcctttgcctgcgctt
ctagtgcttcgatggtggcttgtgcctgcgcttgctgcgcctgtagtgcctgttgagcttcttgtagttgctgttctagctgtgccttggttgccatgctttaagactcta
gtagctttcctgcgatatgtcatgcgcatgcgtagcaaacattgtcctgcaactcattcattatgtgcagtgctcctgttactagtcgtacatactcatatttacctag
tctgcatgcagtgcatgcacatgcagtcatgtcgtgctaatgtgtaaaacatgtacatgcagattgctgggggtgcagggggcggagccaccctgtccatgc
ggggtgtggggcttgccccgccggtacagacagtgagcaccggggcacctagtcgcggataccccccctaggtatcggacacgtaaccctcccatgtcg
atgcaaatctttaacattgagtacgggtaagctggcacgcatagccaagctaggcggccaccaaacaccactaaaaattaatagttcctagacaagaca
aacccccgtgcgagctaccaactcatatgcacgggggccacataacccgaaggggtttcaattgacaaccatagcactagctaagacaacgggcaca
acacccgcacaaactcgcactgcgcaaccccgcacaacatcgggtctaggtaacactgaaatagaagtgaacacctctaaggaaccgcaggtcaatg
agggttctaaggtcactcgcgctagggcgtggcgtaggcaaaacgtcatgtacaagatcaccaatagtaaggctctggcggggtgccataggtggcgca
gggacgaagctgttgcggtgtcctggtcgtctaacggtgcttcgcagtttgagggtctgcaaaactctcactctcgctgggggtcacctctggctgaattggaa
gtcatgggcgaacgccgcattgagctggctattgctactaagaatcacttggcggcgggtggcgcgctcatgatgtttgtgggcactgttcgacacaaccgc
tcacagtcatttgcgcaggttgaagcgggtattaagactgcgtactcttcgatggtgaaaacatctcagtggaagaaagaacgtgcacggtacggggtgga
gcacacctatagtgactatgaggtcacagactcttgggcgaacggttggcacttgcaccgcaacatgctgttgttcttggatcgtccactgtctgacgatgaac
tcaaggcgtttgaggattccatgttttcccgctggtctgctggtgtggttaaggccggtatggacgcgccactgcgtgagcacggggtcaaacttgatcaggtg
tctacctggggtggagacgctgcgaaaatggcaacctacctcgctaagggcatgtctcaggaactgactggctccgctactaaaaccgcgtctaaggggt
cgtacacgccgtttcagatgttggatatgttggccgatcaaagcgacgccggcgaggatatggacgctgttttggtggctcggtggcgtgagtatgaggttgg
ttctaaaaacctgcgttcgtcctggtcacgtggggctaagcgtgctttgggcattgattacatagacgctgatgtacgtcgtgaaatggaagaagaactgtac
aagctcgccggtctggaagcaccggaacgggtcgaatcaacccgcgttgctgttgctttggtgaagcccgatgattggaaactgattcagtctgatttcgcg
gttaggcagtacgttctagattgcgtggataaggctaaggacgtggccgctgcgcaacgtgtcgctaatgaggtgctggcaagtctgggtgtggattccacc
ccgtgcatgatcgttatggatgatgtggacttggacgcggttctgcctactcatggggacgctactaagcgtgatctgaatgcggcggtgttcgcgggtaatg
agcagactattcttcgcacccactaaaagcggcataaaccccgttcgatattttgtgcgatgaatttatggtcaatgtcgcgggggcaaactatgatgggtctt
gttgttgacaatggctgatttcatcaggaatggaactgtcatgctgttatgtgcctggctcctaatcaaagctggggacaatgggttgccccgttgatctgatcta
gttcggattggcggggcttcactgtatctgggggtggcatcgtgaatagattgcacaccgtagtgggcagtgtgcacaccatagtggccatgagcaccacc
acccccagggacgccgacggcgcgaagctctgcgcctggtgcggctcggagatcaagcaatccggcgtcggccggagccgggactactgccgccgc
tcctgccgccagcgggcgtacgaggcccggcgccagcgcgaggcgatcgtgtccgccgtggcgtcggcagtcgctcgccgagatacgtcacgtgacg
aaatgcagcagccttccattccgtcacgtgacgaaactcgggccgcaggtcagagcacggttccgcccgctccggccctgccggacccccggcatccc
gcaagaggcccggcagtaccggcataaccaagcctatgcctacagcatccagggtgacggtgccgaggatgacgatgagcgcattgttagatttcatac
acggtgcctgactgcgttagcaatttaactgtgataaactaccgcattaaagcttatcgatgataagctgtcaaacatggcctgtcgcttgcggtattcggaatc
ttgcacgccctcgctcactgcccgctttccagtcgggaaacctgtcgtgccagctgcattaatgaatcggccaacgcgcggggagaggcggtttgcgtattg
ggcgccagggtggtttttcttttcaccagtgagacgggcaacagctgattgcccttcaccgcctggccctgagagagttgcagcaagcggtccacgctggttt
gccccagcaggcgaaaatcctgtttgatggtggttaacggcgggatataacatgagctgtcttcggtatcgtcgtatcccactaccgagatatccgcaccaa
cgcgcagcccggactcggtaatggcgcgcattgcgcccagcgccatctgatcgttggcaaccagcatcgcagtgggaacgatgccctcattcagcatttg
catggtttgttgaaaaccggacatggcactccagtcgccttcccgttccgctatcggctgaatttgattgcgagtgagatatttatgccagccagccagacgca
gacgcgccgagacagaacttaatgggcccgctaacagcgcgatttgctggtgacccaatgcgaccagatgctccacgcccagtcgcgtaccgtcttcatg
ggagaaaataatactgttgatgggtgtctggtcagagacatcaagaaataacgccggaacattagtgcaggcagcttccacagcaatggcatcctggtca
tccagcggatagttaatgatcagcccactgacgcgttgcgcgagaagattgtgcaccgccgctttacaggcttcgacgccgcttcgttctaccatcgacacc
accacgctggcacccagttgatcggcgcgagatttaatcgccgcgacaatttgcgacggcgcgtgcagggccagactggaggtggcaacgccaatcag
caacgactgtttgcccgccagttgttgtgccacgcggttgggaatgtaattcagctccgccatcgccgcttccactttttcccgcgttttcgcagaaacgtggctg
gcctggttcaccacgcgggaaacggtctgataagagacaccggcatactctgcgacatcgtataacgttactggtttcacattcaccaccctgaattgactct
cttccgggcgctatcatgccataccgcgaaaggttttgcaccattcgatggtgtcaacgtaaatgcatgccgcttcgccttcgcgcgcgaattgcaagctgat
ccgggcttatcgactgcacggtgcaccaatgcttctggcgtcaggcagccatcggaagctgtggtatggctgtgcaggtcgtaaatcactgcataattcgtgt
cgctcaaggcgcactcccgttctggataatgttttttgcgccgacatcataacggttctggcaaatattctgaaatgagctgttgacaattaatcatcggctcgta
taatgtgtggaattgtgagcggataacaatttcacacaggaaacagaattaaaagatatgaccatgattacgccaagcttgcatgcctgcaggtcgac
SEQ ID NO: 7 - CRM197 codon opt2 
ggcgctgatgatgttgttgattcttctaagtctttcgtgatggaaaacttctcttcctaccacggcactaagcctggttacgttgattccattcagaagggtatccag
aagccaaagtctggtacccagggcaactacgacgatgattggaaggagttctactccaccgacaacaagtacgacgctgctggctactctgtagataac
gaaaacccactctctggcaaggctggcggcgtggtcaaggtgacctacccaggcctgaccaaggttctcgcactgaaggtggataacgctgaaactatta
agaaggagctgggtctgtccctcactgaaccactgatggagcaggtcggcaccgaagagttcatcaagcgcttcggtgatggtgcttcccgtgtagtgctct
cccttccattcgctgagggctcctcttccgttgaatacattaacaactgggaacaggctaaggctctgtccgtagaacttgagattaacttcgaaacccgtggc
aagcgtggccaggatgctatgtacgagtacatggctcaggcttgtgcaggcaaccgtgtccgccgctccgtaggttcctccctgtcctgcatcaaccttgatt
gggatgtcatccgcgataagactaagaccaagatcgagtctctgaaggagcacggccctatcaagaacaagatgtccgaatccccaaacaagaccgt
atctgaggaaaaggctaagcagtacctggaagagttccaccagaccgcactggagcaccctgaactgtccgaacttaagaccgttactggcaccaacc
ctgtattcgctggcgctaactacgctgcttgggcagtaaacgttgctcaggttatcgattccgaaaccgctgataacctggaaaagaccactgctgctctttcc
atccttcctggtatcggttccgtaatgggcattgcagacggtgctgttcaccacaacaccgaagagatcgtggcacagtccatcgctctgtcctctctgatggtt
gctcaggctattccactggtaggcgagctggttgatattggtttcgctgcatacaacttcgtagagtccattatcaacctgttccaggtagttcacaactcctaca
accgtccagcttactctccaggccacaagacccagccattccttcacgacggctacgctgtctcctggaacactgttgaagattccatcatccgcactggtttc
cagggcgagtccggccacgacatcaagattactgctgaaaacaccccacttccaatcgctggtgtcctgctgccaactattcctggcaagctggacgttaa
caagtccaagactcacatttccgtaaacggtcgcaagatccgcatgcgttgccgcgctatcgacggcgacgtaacattctgtcgccctaagtctcctgtttac
gttggtaacggtgtgcacgctaaccttcacgtggcattccaccgctcctcctccgagaagattcactctaacgaaatttcctccgattccatcggcgttcttggct
accagaagaccgtagatcacaccaaggttaactctaagctgtccctgttcttcgaaatcaagtcc
SEQ ID NO: 8 - CRM197 codon opt3

ggcgcggatgatgtggtggatagcagcaaaagctttgtgatggaaaactttagcagctatcatggcaccaaaccgggctatgtggatagcattcagaaag
gcattcagaaaccgaaaagcggcacccagggcaactatgatgatgattggaaagaattttatagcaccgataacaaatatgatgcggcgggctatagcg
tggataacgaaaacccgctgagcggcaaagcgggcggcgtggtgaaagtgacctatccgggcctgaccaaagtgctggcgctgaaagtggataacgc
ggaaaccattaaaaaagaactgggcctgagcctgaccgaaccgctgatggaacaggtgggcaccgaagaatttattaaacgctttggcgatggcgcga
gccgcgtggtgctgagcctgccgtttgcggaaggcagcagcagcgtggaatatattaacaactgggaacaggcgaaagcgctgagcgtggaactgga
aattaactttgaaacccgcggcaaacgcggccaggatgcgatgtatgaatatatggcgcaggcgtgcgcgggcaaccgcgtgcgccgcagcgtgggc
agcagcctgagctgcattaacctggattgggatgtgattcgcgataaaaccaaaaccaaaattgaaagcctgaaagaacatggcccgattaaaaacaa
aatgagcgaaagcccgaacaaaaccgtgagcgaagaaaaagcgaaacagtatctggaagaatttcatcagaccgcgctggaacatccggaactga
gcgaactgaaaaccgtgaccggcaccaacccggtgtttgcgggcgcgaactatgcggcgtgggcggtgaacgtggcgcaggtgattgatagcgaaac
cgcggataacctggaaaaaaccaccgcggcgctgagcattctgccgggcattggcagcgtgatgggcattgcggatggcgcggtgcatcataacaccg
aagaaattgtggcgcagagcattgcgctgagcagcctgatggtggcgcaggcgattccgctggtgggcgaactggtggatattggctttgcggcgtataac
tttgtggaaagcattattaacctgtttcaggtggtgcataacagctataaccgcccggcgtatagcccgggccataaaacccagccgtttctgcatgatggct
atgcggtgagctggaacaccgtggaagatagcattattcgcaccggctttcagggcgaaagcggccatgatattaaaattaccgcggaaaacaccccgc
tgccgattgcgggcgtgctgctgccgaccattccgggcaaactggatgtgaacaaaagcaaaacccatattagcgtgaacggccgcaaaattcgcatgc
gctgccgcgcgattgatggcgatgtgaccttttgccgcccgaaaagcccggtgtatgtgggcaacggcgtgcatgcgaacctgcatgtggcgtttcatcgca
gcagcagcgaaaaaattcatagcaacgaaattagcagcgatagcattggcgtgctgggctatcagaaaaccgtggatcataccaaagtgaacagcaa
actgagcctgttttttgaaattaaaagc

## Claims

1. A method for producing a protein in a Corynebacterium cell, wherein the protein is a non-toxic variant of diphtheria toxin and wherein a signal peptide having at least 90% sequence identity to SEQ ID NO: 1 or SEQ ID NO: 5 is fused to the protein.

2. The method according to claim 1, wherein the protein is produced using a DNA molecule having at least 90% sequence identity to SEQ ID NO: 3.

3. A Corynebacterium cell capable of expressing a protein, wherein the protein is a non-toxic variant of diphtheria toxin and wherein a signal peptide having at least 90% sequence identity to SEQ ID NO: 1 or SEQ ID NO: 5 is fused to the protein.

4. The cell according to claim 3, wherein the cell comprises a DNA molecule having at least 90% sequence identity to SEQ ID NO: 3.

5. The method according to claim 1 or 2 the cell according to claim 3 or 4, wherein the Corynebacterium cell is non-pathogenic.

6. The method according to claim 1, 2 or 5 or the cell according to any of claims 3 to 5, wherein the Corynebacterium cell is a *Corynebacterium glutamicum* cell.

7. The method according to any of claims 1, 2, 5 or 6 or the cell according to any of claims 3 to 6, wherein the protein has at least 80% sequence identity to SEQ ID NO: 2.

8. The method according to claim 2 or the cell according to claim 4, wherein the DNA molecule is present in the Corynebacterium cell in the form of a plasmid or at a genomic locus.

9. The method according to any of claims 1, 2 or 5 to 8 or the cell according to any of claims 3 to 8, wherein the protein is secreted by the Corynebacterium cell or the Corynebacterium cell is capable of secreting the protein.

10. The method or the cell according to claim 9, wherein the protein is secreted into the extracytosolic space.

11. The method or the cell according to any of claims 9 or 10, wherein the signal peptide is cleaved from the protein during secretion.

12. A method for producing a Corynebacterium cell according to any of claims 3 to 11.

13. The method according to claim 12, wherein a sensor is used to identify a Corynebacterium cell showing high expression of the non-toxic variant of diphtheria toxin.

14. The method according to claim 13, wherein the sensor is a fluorescent protein under the control of at least one heterologous promoter, which, in the wild type of the cell, controls the expression of a gene of which the expression in the wild-type cell depends on the mount of protein that is secreted across the cytoplasmic membrane into the extracytosolic space, so that the expression of the fluorescent protein depends on the amount of protein that is secreted across the cytoplasmic membrane into the extracytosolic space.

15. A DNA molecule encoding a non-toxic variant of diphtheria toxin, the DNA molecule comprising a part having at least 90% sequence identity to SEQ ID NO: 4 or SEQ ID NO: 6.
